# EUROPEAN PATENT APPLICATION

(11) **EP 3 182 130 A1**
(43) Date of publication of application: **21.06.2017**
(21) Application number: 15832399.8
(22) Date of filing: 13.08.2015
(51) Int. Cl.: G01N 33/68, G01N 33/53

(54) **METHOD AND KIT FOR DETERMINING RISK OF PRETERM BIRTH AND/OR BIRTH OF LOW-BIRTH-WEIGHT BABY**

(30) Priority: 14.08.2014 JP 2014165280
(71) Applicant: Hiroshima University, Higashi-Hiroshima-shi Hiroshima 739-8511 (JP)
(72) Inventor: TAKATA, Takashi, Hiroshima-shi Hiroshima 734-8553 (JP); MIYAUCHI, Mutsumi, Hiroshima-shi Hiroshima 734-8553 (JP); KUDO, Yoshiki, Hiroshima-shi Hiroshima 734-8553 (JP)
(74) Representative: Boult Wade Tennant
(86) International application number: PCT/JP2015/072928
(87) International publication number: WO 2016/024627

(57) **Abstract**

The present application relates to a method for determining a risk of preterm birth and/or low birth weight of a pregnant female comprising measuring a galectin-3 (Gal-3) level in a blood, plasma, or serum sample obtained from the pregnant female, or a kit or marker which may be used in the method. The present application also relates to a medicament effective to reduce a risk of preterm birth and/or low birth weight, a pharmaceutical composition comprising the medicament, or a method for reducing a risk of preterm birth and/or low birth weight of a pregnant female comprising administering the medicament or the pharmaceutical composition to the pregnant female.

## Description

### TECHNICAL FIELD

This application claims the benefit of priority of the prior Japanese patent application (Japanese Patent Application No. 2014-165280), the entire contents of which are incorporated herein by reference.

The disclosure relates to a method for determining a risk of preterm birth and/or low birth weight of a pregnant female comprising measuring a galectin-3 (Gal-3) level in a blood, plasma, or serum sample obtained from the pregnant female, or a kit or a marker which may be used in the method. Alternatively, the disclosure relates to a medicament for reducing a risk of preterm birth and/or low birth weight, a pharmaceutical composition comprising the medicament, or a method for reducing a risk of preterm birth and/or low birth weight of a pregnant female comprising administering the medicament or the pharmaceutical composition to the pregnant female.

### BACKGROUND ART

Preterm birth of a low birth weight infant is the most common cause of morbidity and mortality in newborn infants. An epidemiologic study in 2010 estimated that 11.1% of all births in the world was preterm. The World Health Organization (WHO) has aimed to reduce the number of preterm birth, which is associated with 75% of perinatal mortality and more than half of the long-term morbidity of diseases such as cerebral palsy and bronchopulmonary dysplasia. However, even a method for determining a risk of preterm birth has not been established. Merely a possible cause of preterm birth, chorioamnionitis, was suggested.

Periodontitis is a mild persistent chronic inflammatory disease caused by infection of periodontopathic bacteria, the prevalence of which is up to 80%. It has been revealed that the periodontopathic bacteria or factors derived therefrom enter the blood circulation and are disseminated through the whole body to affect systemic diseases such as diabetes, cardiovascular diseases, arteriosclerosis, or osteoporosis.

Researchers including the inventors have succeeded in establishing a new odontogenic infected mouse model by inoculating the pulp chambers of the molars with a periodontopathic bacterium, *Porphyromonas gingivalis* (*P*.*g*.), and inducing periapical granuloma which serves as a persistent source of *P.g.* (Non-Patent Literature 1). In the model mice *P.g.* was frequently detected in the placenta and rate of preterm birth and low birth weight was significantly higher than that in uninfected mice (Non-Patent Literatures 2 and 3).

During the investigation of the immunological phenomena in the infected placenta, the inventors used an anti-Gal-3 antibody for detecting macrophages. Interestingly, the expression of Gal-3 was found remarkably increased in various placental cells (Non-patent Literatures 2 and 3). Gal-3, a member of the β-galactoside binding lectin family, is an immunomodulatory factor that plays an important role both in the proinflammatory and the antiinflammatory processes (Non-patent Literature 4). Gal-3 was also significantly increased in the amniotic fluid of the model mice (Non-patent Literatures 2 and 3). However, the serum Gal-3 level of the maternal mice has not been disclosed.

Demmert, M. et al. studied the expression of Gal-3 in cord blood of human infants. They found that there was a positive correlation between the length of the gestation and the Gal-3 level in the cord blood and that the expression of Gal-3 was significantly higher in the cord blood of preterm infants compared to that of full-term infants (Non-patent Literature 5). However, no correlation has been reported between the serum level of Gal-3 in pregnant women and preterm birth.

In summary, the correlation between preterm birth and the expression level of Gal-3 in the placenta, the amniotic fluid or the cord blood has been suggested. However, because sampling the placenta, the amniotic fluid or the cord blood from a pregnant woman may hurt the fetus, the correlation cannot be clinically used for determining the risk of preterm birth.

### REFERENCES

### NON-PATENT LITERATURE

[Non-Patent Literature 1] Furusho, H., et al., Dental infection of Porphyromonas gingivalis exacerbates high fat diet-induced steatohepatitis in mice. J Gastroenterol, November 2013, 48 (11), pp 1259-1270
[Non-Patent Literature 2] Ao M, et al., Odontogenic infection of P. gingivalis induces preterm delivery through Galectin-3, Abstract for International Association of Dental Research General Session (Seattle), March 20-23, 2013
[Non-Patent Literature 3] Ao M, et al., Role of Galectin-3 in preterm delivery induced by odontogenic infection of Porphyromonas gingivalis, Abstract for 56th Annual Meeting in Spring, May 31- June 1, 2013 in Tokyo, the Japanese Society of Periodontology
[Non-Patent Literature 4] Dumic, J., S. Dabelic, and M. Flogel. 2006. Galectin-3: an open-ended story. In Biochim Biophys Acta. Netherlands. 616-635
[Non-Patent Literature 5] Demmert M, et al., Galectin-3 in cord blood of term and preterm infants. Clin Exp Immunol. 2012, 167(2):246-51

### SUMMARY OF THE INVENTION

The inventors found that the serum level of Gal-3 was higher in pregnant females with preterm birth than in pregnant females with full-term birth.

In an aspect, provided is a method for determining a risk of preterm birth and/or low birth weight of a pregnant female comprising measuring a Gal-3 level in a blood, plasma, or serum sample obtained from the pregnant female.

In an aspect, provided is a kit for determining a risk of preterm birth and/or low birth weight of a pregnant female comprising an anti-Gal-3 antibody for measuring a Gal-3 level in a blood, plasma, or serum sample obtained from the pregnant female.

In an aspect, provided is Gal-3 in blood, plasma, or serum as a marker of a risk of preterm birth and/or low birth weight of a pregnant female. In another aspect, provided is use of Gal-3 in a blood, plasma, or serum sample obtained from a pregnant female as a marker of a risk of preterm birth and/or low birth weight of the pregnant female.

In an aspect, provided is a pharmaceutical composition for reducing a risk of preterm birth and/or low birth weight comprising a Gal-3 inhibitor as an active ingredient.

In an aspect, provided is a method for reducing a risk of preterm birth and/or low birth weight of a pregnant female comprising administering an effective amount of a Gal-3 inhibitor to the pregnant female. In another aspect, provided is a method for reducing a risk of preterm birth and/or low birth weight comprising administering a pharmaceutical composition comprising a Gal-3 inhibitor as an active ingredient to a pregnant female.

The method for determining a risk of preterm birth and/or low birth weight of a pregnant female employs the simple step to measure the Gal-3 level in the blood, plasma, or serum sample and does not require amniocentesis, which may adversely affect the pregnant female and the fetus. Referring to the result, pregnant females determined to have the risk of preterm birth and/or low birth weight can be preferentially treated for preventing preterm birth and/or low birth weight. Such selection will reduce the number of preterm birth and/or low birth weight efficiently. Furthermore, the inhibition of Gal-3 may reduce the risk of preterm birth and/or low birth weight.

### BRIEF DESCRIPTION OF DRAWINGS

Fig. 1 shows gestational days (gd) (A) and birth weight (B) of mice in a negative control (NC) group and a *P.g.-*infected group.
Fig. 2 shows immunohistochemical staining indicating localization of *P.g.* in a placental tissue of a mouse in NC group and P.g.-infected group.
Fig. 3 shows immunohistochemical staining indicating localization of Gal-3 in a placental tissue of a mouse in NC group and P.g.-infected group.
Fig. 4 shows Gal-3 levels in amniotic fluid (A) and serum (B) of mice in NC group and P.g.-infected group.
Fig. 5 shows protein concentrations (upper graph) and gene expression levels (lower electropherogram) of Gal-3 in a human trophoblast cell line (HTR-8) stimulated by *P.g.*-LPS.
Fig. 6 shows Gal-3 protein levels in HTR-8 stimulated by TNF-α.
Fig. 7A shows serum Gal-3 levels in pregnant women with full-term birth and preterm birth. The bar graph represents the average Gal-3 levels at each gestational age.
Fig. 7B shows serum Gal-3 levels in pregnant women with full-term birth and preterm birth. Each Gal-3 level are plotted against the gestational age.
Fig. 7C shows serum Gal-3 levels in pregnant women with full-term birth and preterm birth. The Gal-3 levels in pregnant women with full-term birth at the gestational age of 38 weeks and the Gal-3 levels in pregnant women with preterm birth immediately before the delivery are plotted.
Fig. 8 is the bar graph showing average serum Gal-3 levels in pregnant women of each gestational age.
Fig. 9 shows gestational days of mice in a NC group, a *P.g.* group, and a *P.g*./N-acetyllactosamine (N-Lac) group.
Fig. 10 shows immunohistochemical staining indicating localization of TNF-α, COX-2, and Gal-3 in a placental tissue of a mouse in NC group, the *P.g.* group, and the *P.g*./N-Lac group.
Fig. 11 shows protein levels of TNF-α and Gal-3 in HTR-8 in the presence of P.g.-LPS and/or N-Lac.

### DETAILED DESCRIPTION

Unless defined otherwise, the terms used herein have the meaning as commonly understood to those skilled in the art in the fields including organic chemistry, medicine, pharmacology, molecular biology, and microbiology. Definitions of several terms used herein are described below. The definitions herein take precedence over the general understanding.

As used herein "preterm birth" means a delivery with a gestational period shorter than that of full-term birth. Regarding human, preterm birth means a delivery at the gestational age not less than 22 weeks and 0 day and not more than 36 weeks and 6 days, wherein a delivery at the gestational age not more than 31 weeks and 6 days is further referred to as very preterm birth. In human full-term birth means a delivery at the gestational age not less than 37 weeks and 0 day and not more than 41 weeks and 6 days. For calculating the gestational age in human, the beginning of the last menstrual period is estimated as day 0, provided that the menstrual cycle is about 28 days. When the menstrual cycle is not normal, the gestational age is deduced from the size of the embryo or fetus in the early pregnancy. Even if the menstrual cycle is normal, the gestational age may be based on the size of the embryo or fetus. If the timing of the ovulation can be estimated with a high degree of accuracy, the gestational age at the time of the ovulation can be estimated as 2 weeks and 0 day.

As used herein "low birth weight" means a delivery where the weight of the infant at birth is lighter than the standard birth weight inherently defined for the species. As for human, "low birth weight" means a delivery where the birth weight is less than 2500 g.

In an embodiment, the pregnant female is a pregnant woman. For example, the pregnant woman is at the gestational age not more than 36 weeks and 6 days. The pregnant woman may be a possible pregnant woman who has undergone treatment for infertility and the risk of preterm birth and/or low birth weight may be determined before the pregnancy is confirmed.

In an embodiment, the pregnant female has a risk factor of preterm birth and/or low birth weight. Risk factors of preterm birth and/or low birth weight include any risk factors known in the art, for example, multiple pregnancy, previous preterm birth or miscarriage, lack of prenatal care, poor nutrition, stress, early or advanced maternal age pregnancy, smoking, diabetes, high blood pressure, infectious diseases, and uterine or cervical problems.

In an embodiment, the pregnant female is suffered from periodontitis. Causative bacteria of periodontitis include *Porphyromonas gingivalis, Prevotella intermedia, Aggregatibacter actinomycetemcomitans, Treponema denticola,* and *Tannerella forsythensis.* For example, the pregnant female is suffered from periodontitis caused by *Porphyromonas gingivalis.*

The blood sample may be obtained in a conventional manner, for example from vein or artery of the pregnant female. The plasma or serum sample may be prepared from the blood sample by any suitable methods known to those skilled in the art. The methods are not limited and may include any clinically acceptable steps such as addition of an anticoagulant and centrifugation. Prior to measuring the Gal-3 level, the blood, plasma or serum sample may be stored at a low temperature, for example in a freezer, during or after the preparation of the sample.

The Gal-3 level may be measured by any methods known in the art. For example, the Gal-3 level may be measured by a test using biospecific affinity. Tests using biospecific affinity are known to those skilled in the art and include an immunoassay without limitation. For example, immunoassays include competitive or noncompetitive assay systems such as western blotting, radioimmunoassay, ELISA (Enzyme-Linked ImmunoSorbent Assay, including sandwich ELISA, competitive ELISA, and direct ELISA), immunoprecipitation, precipitation reaction, immunodiffusion, immune agglutination assay, complement fixation test, immunoradiometric assay, fluorescent immunoassay, and protein A immunoassay.

In the immunoassays, an anti-Gal-3 antibody may be employed. The Gal-3 level is measured by contacting a blood, plasma, or serum sample obtained from the pregnant female to an anti-Gal-3 antibody under the condition where Gal-3 and the anti-Gal-3 antibody can form an immunocomplex. Those skilled in the art may select a suitable anti-Gal-3 antibody.

As used herein, "antibody binding to Gal-3" or "anti-Gal-3 antibody" may be an antibody capable of binding to Gal-3, a fragment thereof or a derivative thereof, i.e., an affinity ligand based on an immunoglobulin scaffold. The antibody may be a monoclonal or polyclonal antibody of any origins including mouse, rat, rabbit, goat, and human. The antibody may be a chimeric antibody comprising sequences from different species, such as a partly humanized antibody, e.g., a partly humanized mouse antibody. The polyclonal antibodies may be produced by immunization of animals with the antigen. The monoclonal antibodies can be produced using the hybridoma technology. The antibody fragments and derivatives are capable of selective interaction with Gal-3 and include, for example, Fab fragments consisting of the first constant domain of the heavy chain (CH1), the constant domain of the light chain (CL), the variable domain of the heavy chain (VH) and the variable domain of the light chain (VL) of an intact immunoglobulin protein; Fv fragments consisting of the two variable antibody domains VH and VL; single chain Fv fragments (scFv) consisting of the two VH and VL domains linked together by a flexible peptide linker; camelid heavy-chain dimers and single variable domains, and single domain scaffolds like e.g., the New Antigen Receptor (NAR) from the nurse shark and minibodies based on a variable heavy domain.

The anti-Gal-3 antibody may be labeled. Those skilled in the art may select a suitable label, non-limiting examples of which include fluorescent dyes or metals (e.g., fluorescein, rhodamine, phycoerythrin, fluorescamine), chromophoric dyes (e.g., rhodopsin), chemiluminescent compounds (e.g., luminal, imidazole), bioluminescent proteins (e.g., luciferin, luciferase), haptens (e.g., biotin), enzymes (e.g., peroxidase, alkaline phosphatase, betalactamase), radioisotopes (e.g., ³H, ¹⁴C, ³²P, ³⁵S, ¹²⁵I) and particles (e.g., particles of metal such as gold), and fluorescent semiconductor nanocrystals (quantum dots). The different types of the labels can be conjugated to the anti-Gal-3 antibody using various chemistries known to those skilled in the art, e.g., the amine reaction or the thiol reaction; other reactive groups, e.g., aldehydes, carboxylic acids and glutamine may also be used.

The methods of detection of the labels may include, but is not limited to, fluorometric, luminometric and enzymatic techniques. Fluorescent labels are detected and/or quantified by exposing the fluorescent labels to light of a specific wavelength and thereafter detecting and/or quantifying the emitted light in a specific wavelength region. Luminescent labels may be detected and/or quantified by detecting and/or quantifying luminescence developed during a chemical reaction. Enzymatic labels are detected and/or quantified by detecting and/or quantifying a color shift in the sample arising from a chemical reaction. ELISA is an example of methods based on an enzymatic reaction. Those skilled in the art can modify protocols for proper detection and/or quantification.

The steps of each immunoassay are known to those skilled in the art. An example is as follows. A capture antibody is attached to a solid phase, for example a plastic tube, a microplate, a glass bead, or a substrate. Examples of the capture antibody include anti-Gal-3 antibodies capable of binding to an epitope that is different from the epitope recognized by the labeled anti-Gal-3 antibody mentioned above, fragments thereof and analogues thereof, e.g. anti-Gal-3 polyclonal and monoclonal antibodies. Those skilled in the art can use the capture antibody at a suitable concentration, for example, in the range of 1 to 10 µg/mL, e.g. at 2 or 5 µg/mL.

The solid phase coated with the capture antibody may be blocked with proteins that are not involved in antigen-antibody reactions or enzymatic reactions. Examples of the protein suitable for the blocking include skim milk, albumin, fish gelatin, and casein. The protein for the blocking may be used at a concentration, for example, in the range of 0.1 to 5% or 0.5 to 2%, e.g. at 1%.

The capture antibody on the solid phase is contacted with a blood, plasma, or serum sample obtained from a pregnant female, and is incubated under the condition where the capture antibody and Gal-3 in the sample can form an immunocomplex. The sample may be diluted before the contact. Those skilled in the art can dilute the sample at a suitable rate, considering the type of the sample or other conditions, for example, in the range of 1 to 20-fold or 5 to 10-fold, e.g. at 5-fold or 10-fold. Alternatively, the sample may be used without dilution.

The solid phase is washed, added with the labeled anti-Gal-3 antibody as mentioned above, and then incubated under the condition where Gal-3 bound to the capture antibody and the labeled anti-Gal-3 antibody can form an immunocomplex. Those skilled in the art can use the labeled anti-Gal-3 antibody at a suitable concentration, considering the type of the sample or other conditions, for example, in the range of 0.1 to 10 µg/mL or 0.1 to 2 µg/mL, e.g. at 0.1 µg/mL.

The solid phase is washed again, signals from the labeled anti-Gal-3 antibody bound to Gal-3 are detected and quantified by a technique such as a fluorometric, luminometric or enzymatic technique mentioned above. The measurement of the signal correlates to the Gal-3 level in the blood, plasma, or serum sample.

Alternatively, an unlabeled anti-Gal-3 antibody may be used for detecting Gal-3 bound to the capture antibody. In this case a labeled secondary antibody that recognizes the unlabeled detecting anti-Gal-3 antibody may be used. The secondary antibody may be labeled and detected in the same manner as the labeled anti-Gal-3 antibody mentioned above.

In an embodiment, Gal-3 levels in two or more blood, plasma, or serum samples obtained from a pregnant female at different time points are measured and compared. For more precise comparison, preferably the samples are prepared and stored under the same conditions and the Gal-3 levels are measured by the same method. The samples may be obtained two or more times, e.g. 2 to 10 times or more, and each Gal-3 level may be measured. The intervals of the sample collections and measurements may be constant or not. For example, the interval may be one, two, three or five days, one or two weeks, or one, two, or three months.

In this embodiment, the risk of preterm birth and/or low birth weight of the pregnant female is determined to have increased when the Gal-3 level in the sample obtained later is higher than the Gal-3 level in the sample obtained earlier. Similarly, the risk of preterm birth and/or low birth weight of the pregnant female is determined to have decreased when the Gal-3 level in the sample obtained later is lower than the Gal-3 level in the sample obtained earlier.

In an embodiment, a Gal-3 level in a blood, plasma, or serum sample obtained from a pregnant female is compared with a reference value. The reference value may be determined by measuring Gal-3 levels in a group of pregnant females with full-term birth and in a group of pregnant females with preterm birth and comparing them.

For example, the reference value may be determined on the basis of the average value of Gal-3 levels at the time of delivery in a group of pregnant females with full-term birth, e.g. pregnant women who delivered at the gestational age not less than 37 weeks and 0 day and not more than 41 weeks and 6 days. In this embodiment, the pregnant woman is determined to have a risk of preterm birth and/or low birth weight when the Gal-3 level in the sample is higher than the reference value. Similarly, the pregnant woman is determined not to have a risk of preterm birth and/or low birth weight when the Gal-3 level in the sample is lower than the reference value.

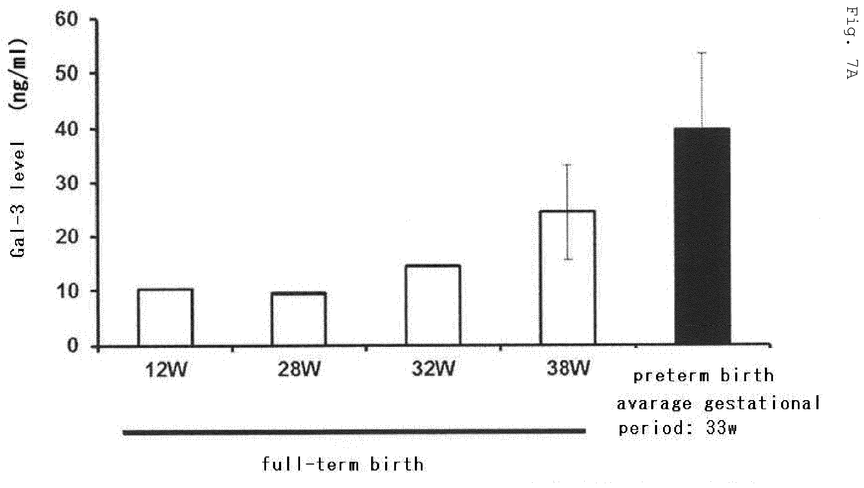

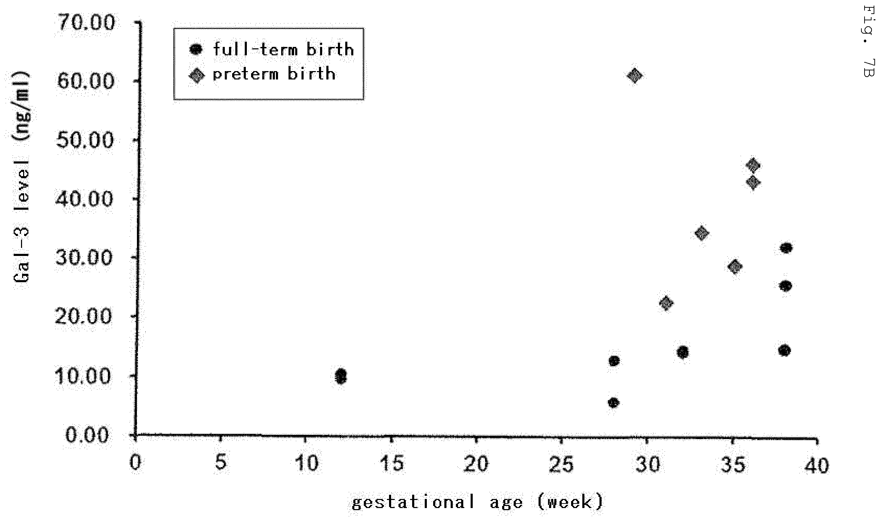

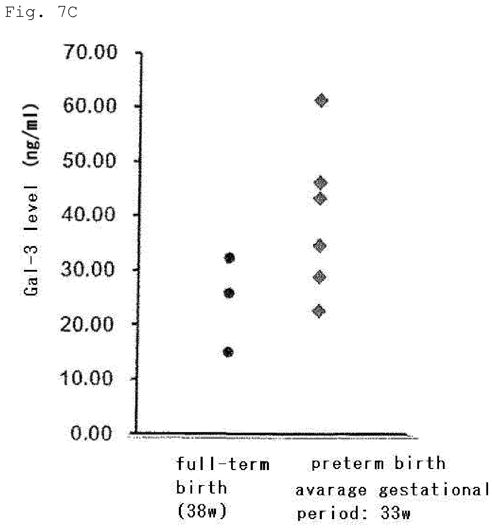

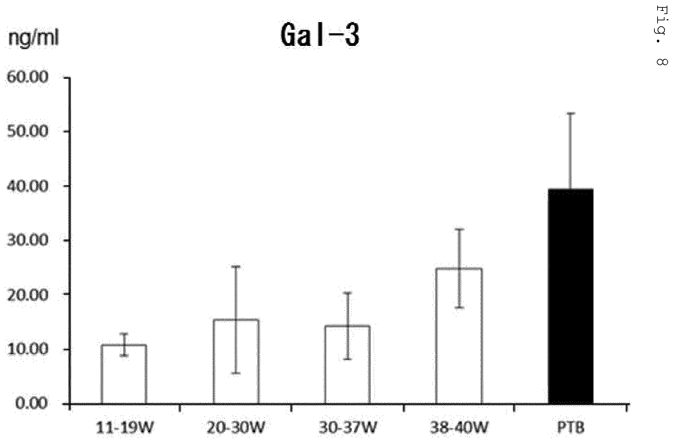

For example, the reference value may be determined on the basis of the data shown in Table 4 in Example 9 and Figs. 7 and 8. For example, the reference value may be calculated according to the formula: "average value of serum Gal-3 levels in a full-term birth group" minus (-) "standard deviation (σ) thereof". For example, in Example 9, the reference value is based on the Gal-3 levels measured at the gestational age between 38 weeks and 40 weeks in the full-term birth group, being about 18 (17.6) in accordance with the formula. When a Gal-3 level of a pregnant woman at a gestational age not more than 36 weeks and 6 days is not less than 18 ng/ml, the pregnant woman may be determined to have a high risk of preterm birth and/or low birth weight. Nevertheless, the reference value may be corrected, e.g. in view of actual conditions of the measurement. For example, when a Gal-3 level in a pregnant woman at a gestational age not more than 36 weeks and 6 days is not less than the corrected reference value, e.g. 13 ng/ml or 15 ng/ml, the pregnant woman may be determined to have a risk of preterm birth and/or low birth weight even if the measured Gal-3 level is lower than the original reference value, 18 ng/ml. In another example, the reference value may be the average value of serum Gal-3 levels in a full-term birth group. For instance, in Example 9, the average value of the Gal-3 levels measured at the gestational age between 38 weeks and 40 weeks in the full-term birth group is about 25 (24.9). When a Gal-3 level of a pregnant woman at a gestational age not more than 36 weeks and 6 days is not less than 25 ng/ml, the pregnant woman may be determined to have a very high risk of preterm birth and/or low birth weight.

In an embodiment, a pregnant female who has been determined to have a risk of preterm birth and/or low birth weight may be treated for preventing preterm birth and/or low birth weight. For preventing preterm birth and/or low birth weight, the pregnant female may be rested. Furthermore, the pregnant female may be administered with a medicament, for example, a tocolytic agent such as ritodrine hydrochloride, isoxsuprine hydrochloride, or magnesium sulfate, or a Gal-3 inhibitor such as N-acetyllactosamine as mentioned below.

In an aspect, a kit for determining a risk of preterm birth and/or low birth weight of a pregnant female comprising an anti-Gal-3 antibody for measuring a Gal-3 level in a blood, plasma, or serum sample obtained from the pregnant female is provided. The anti-Gal-3 antibody may be those as mentioned above. The anti-Gal-3 antibody may be labeled with a label as mentioned above. For example, an anti-Gal-3 antibody labeled with peroxidase may be employed. The kit may be used in the method for determining a risk of preterm birth and/or low birth weight of a pregnant female as mentioned above.

The kit may comprise a capture antibody which may be an anti-Gal-3 antibody, a fragment thereof or a derivative thereof as mentioned above. The capture antibody may be provided as an antibody solution of a desired concentration mentioned above or may be attached to a solid phase such as a plastic tube, a microplate, a glass bead, or a substrate.

In an aspect, a marker of a risk of preterm birth and/or low birth weight of a pregnant female is provided, wherein the marker is Gal-3 in blood, plasma, or serum. Because the level of the marker increases as the risk of preterm birth and/or low birth weight increases, the level may be used for determining the risk of preterm birth and/or low birth weight. In another aspect, use of Gal-3 in a blood, plasma, or serum sample obtained from a pregnant woman as a marker of a risk of preterm birth and/or low birth weight of the pregnant female is provided.

In an aspect, a pharmaceutical composition for reducing risk of preterm birth and/or low birth weight comprising a Gal-3 inhibitor as an active ingredient is provided.

In an aspect, use of a Gal-3 inhibitor for reducing a risk of preterm birth and/or low birth weight is provided.

In an aspect, use of a Gal-3 inhibitor for manufacturing a pharmaceutical composition for reducing a risk of preterm birth and/or low birth weight is provided.

In an aspect, a method for reducing a risk of preterm birth and/or low birth weight of a pregnant female comprising administering an effective amount of a Gal-3 inhibitor or a pharmaceutical composition comprising a Gal-3 inhibitor as an active ingredient to the pregnant female is provided.

As used herein, "reducing a risk of preterm birth and/or low birth weight" means preventing preterm birth and/or low birth weight, extending the gestational period, delaying the delivery, or promoting the full-term birth in a pregnant female, for example, a pregnant woman at gestational age not more than 36 weeks and 6 days.

The pregnant female to be treated may be a pregnant female who has been determined to have a risk of preterm birth and/or low birth weight for example by the method mentioned above. The pregnant female may be a pregnant female having a risk factor of preterm birth and/or low birth weight. Risk factors of preterm birth and/or low birth weight include any risk factors known in the art, for example, multiple pregnancy, previous preterm birth or miscarriage, lack of prenatal care, poor nutrition, stress, early or advanced maternal age pregnancy, smoking, diabetes, high blood pressure, infectious diseases, and uterine or cervical problems.

As used herein, "Gal-3 inhibitor" may be any substances that inhibit or reduce activity of Gal-3. For example, the Gal-3 inhibitor is a compound of formula (I): 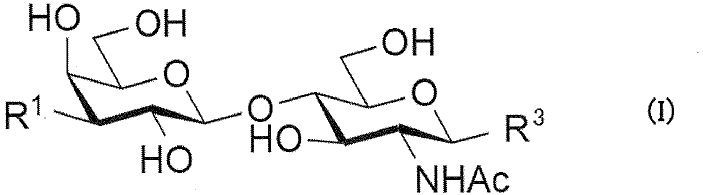 wherein
R¹ is -OH, amino or -NH-X-R²,
X is -C(O)- or -S(O)₂-,
R² is alkyl, alkenyl, wherein the alkyl and alkenyl may be optionally substituted with one or more substituents selected from the group consisting of halogen, -COOH and amino; phenyl optionally substituted with one to five substituents selected from the group consisting of halogen, alkyl, alkoxy, -COOH and nitro; or amino optionally substituted with phenyl, and R³ is -OH or methoxy,
or an ester, pharmaceutically acceptable salt or solvate thereof.

As used herein "alkyl" refers to a monovalent saturated aliphatic hydrocarbyl group having from 1 to 10, for example 1 to 6 carbon atoms. The term "C_{x-y}alkyl" refers to an alkyl group having from x to y carbon atoms. For example, alkyl refers to, but is not limited to, a linear or branched hydrocarbyl group such as methyl (CH₃-), ethyl (CH₃CH₂-), n-propyl (CH₃CH₂CH₂-), isopropyl ((CH₃)₂CH-), n-butyl (CH₃CH₂CH₂CH₂-), isobutyl ((CH₃)₂CHCH₂-), sec-butyl ((CH₃) (CH₃CH₂)CH-), t-butyl ((CH₃)₃C-), n-pentyl (CH₃CH₂CH₂CH₂CH₂-), or neopentyl ((CH₃)₃CCH₂-).

As used herein "alkenyl" refers to a hydrocarbyl group having from 2 to 6, for example 2 to 4 carbon atoms and having at least one, for example one or two carbon-carbon double bonds (C=C). For example, alkenyl refers to, but is not limited to, vinyl, allyl, or but-3-en-yl.

As used herein "alkoxy" refers to the group -O-alkyl in which alkyl is as defined above. For example, alkoxy refers to, but is not limited to, methoxy, ethoxy, n-propoxy, isopropoxy, n-butoxy, t-butoxy, sec-butoxy, or n-pentoxy.

As used herein "amino" refers to the group -NH₂.
As used herein "nitro" refers to the group -NO₂.
As used herein "halogen" refers to fluoro, chloro, bromo, or iodo.

As used herein "compound" refers to a compound encompassed by formula (I) disclosed herein and optionally indicates an ester, pharmaceutically acceptable salt or solvate thereof. The term optionally indicates a stereoisomer or tautomer of the compound.

The compounds disclosed herein may, depending on the substitution pattern, exist in stereoisomeric forms which either are related as image and mirror image (enantiomers) or are not related as image and mirror image (diastereomers). Both the enantiomers or diastereomers and respective mixtures thereof are included. The racemic forms can, just like the diastereomers, be separated in a known manner into the stereoisomerically pure constituents. Certain compounds may also exist in tautomeric forms. This is known to those skilled in the art, and such compounds are likewise included.

The pharmaceutically acceptable, i.e. physiologically acceptable, salt may be a salt of the compound disclosed herein with an inorganic or organic acid. Preferred salts are those with an inorganic acid such as, for example, hydrochloric acid, hydrobromic acid, phosphoric acid or sulfuric acid, or salts with an organic carboxylic or sulfonic acid such as, for example, acetic acid, trifluoroacetic acid, propionic acid, maleic acid, fumaric acid, malic acid, citric acid, tartaric acid, lactic acid, or benzoic acid, or methanesulfonic acid, ethanesulfonic acid, benzenesulfonic acid, toluenesulfonic acid or naphthalenedisulfonic acid.

The pharmaceutically acceptable salt which may also be mentioned is a salt with a conventional base, such as, for example, an alkali metal salt (e.g. sodium or potassium salt), an alkaline earth metal salt (e.g. calcium or magnesium salt) or an ammonium salt derived from ammonia or an organic amine such as, for example, diethylamine, triethylamine, ethyldiisopropylamine, procaine, dibenzylamine, N-methylmorpholine, dihydroabietylamine or methylpiperidine.

As used herein "solvate" refers to a form of the compound disclosed herein which forms a complex in the solid or liquid state through coordination with a solvent molecule. A hydrate is a specific form of the solvate in which the coordination takes place with water.

As used herein "ester" refers to an ester that hydrolyzes in vivo and includes those that break down readily in a human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety has, for example, not more than six carbon atoms. Examples of particular esters include formates, acetates, propionates, butyrates, acrylates and ethylsuccinates.

In an embodiment, the compound of formula (I) wherein
R¹ is -OH, amino or -NH-X-R², and
X-R² is -C(O)-CH₃, -S(O)₂-CH₃, 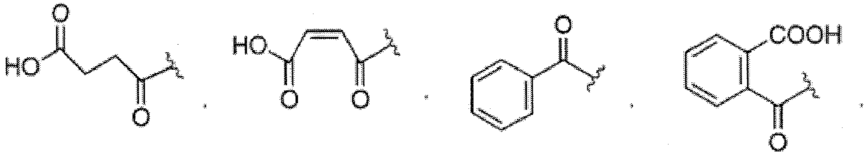 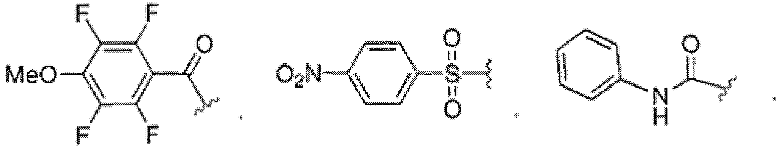 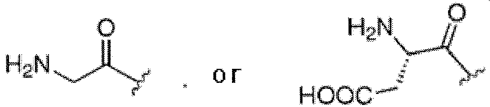 or an ester, pharmaceutically acceptable salt or solvate thereof may be used.

In an embodiment, the compound of formula (I) wherein
R¹ is -OH or -NH-X-R², and
X-R² is 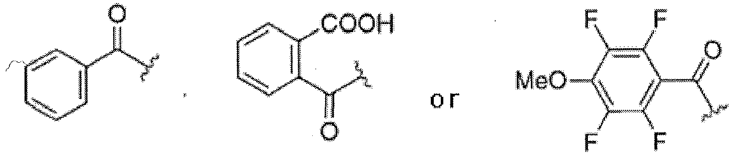 or an ester, pharmaceutically acceptable salt or solvate thereof may be used.

In an embodiment, N-acetyllactosamine represented by the following formula 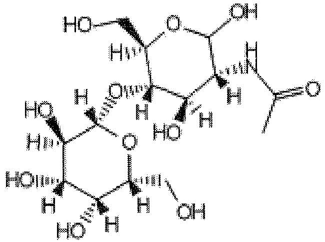 or an ester, pharmaceutically acceptable salt or solvate thereof may be used.

Those skilled in the art can prepare the compound of formula (I), or an ester, pharmaceutically acceptable salt or solvate thereof with reference to Soerme P. et al., "Low micromolar inhibitors of galectin-3 based on 3'-derivatization of N-acetyllactosamine", ChemBioChem, 2002, 3, 183-189. Those skilled in the art also can test the compound of formula (I), or an ester, pharmaceutically acceptable salt or solvate thereof for its activity to inhibit Gal-3.

As used herein, "pharmaceutical composition" means a pharmaceutical composition comprising one or more Gal-3 inhibitors together with one or more nontoxic, inert pharmaceutically acceptable auxiliaries and/or carriers. The pharmaceutical composition may be prepared by a known conventional method, for example, by mixing a Gal-3 inhibitor with an auxiliary and/or carrier.

In an embodiment, the pharmaceutical composition may contain, for example, from about 0.01% to about 99.9%, from about 0.5% to about 50%, or from about 2.5% to about 25%, by weight of the composition, of the Gal-3 inhibitor.

The effective amount of the Gal-3 inhibitor is generally from about 0.1 to about 10 mg/kg body weight, preferably from about 0.5 to about 5 mg/kg body weight, per 24 hours.

In spite of this, it may be necessary to deviate from the amounts mentioned, namely depending on body weight, administration route, individual response to the active ingredient, the type of preparation and the time or the interval at which administration takes place. Thus, in some cases it may be sufficient to administer less than the abovementioned minimum amount, whereas in other cases the upper limit mentioned has to be exceeded. In the case of the administration of relatively large amounts, it may be expedient to divide these into a plurality of individual doses which are administered over the course of the day.

The Gal-3 inhibitor can act systemically and/or locally. For this purpose, it may be administered in a suitable manner, such as, for example, orally, parenterally, pulmonally, nasally, sublingually, rectally, dermally, transdermally, transvaginally, or as an implant or stent. For these administration routes, the active ingredient may be administered in suitable administration forms.

Suitable for oral administration are administration forms which work in accordance with the prior art and release the Gal-3 inhibitor rapidly and/or in modified form and which comprise the Gal-3 inhibitor in crystalline and/or amorphicized and/or dissolved form, such as, for example, tablets (uncoated or coated tablets, for example with enteric coats or coats which dissolve in a delayed manner or are insoluble and which control the release of the active ingredient), films/wafers or tablets which dissolve rapidly in the oral cavity, films/lyophilizates, capsules (for example hard or soft gelatin capsules), sugar-coated tablets, granules, pellets, powders, emulsions, suspensions, aerosols or solutions.

Parenteral administration may take place by circumventing a bioabsorption step (for example intravenously, intraarterially, intracardially, intraspinally or intralumbarly), or with bioabsorption (for example intraarticularly, intramuscularly, subcutaneously, intracutaneously, percutaneously, transvaginally, intraperitoneally, or via inhalation). Administration forms suitable for the parenteral administration are inter alia preparations for injection or infusion in the form of solutions, suspensions, emulsions, lyophilizates or sterile powders.

Suitable for other administration routes are, for example, medicaments suitable for inhalation (inter alia powder inhalers, nebulizers), nose drops, solutions or sprays; tablets to be administered lingually, films/wafers or capsules, suppositories, vaginal capsules, aqueous suspensions (lotions, shaking mixtures), lipophilic suspensions, ointments, creams, transdermal therapeutic systems (for example patches), milk, pastes, foams, powders for pouring, implants or stents.

The Gal-3 inhibitor can be converted into the administration forms mentioned. This can be carried out in a manner known per se by mixing the Gal-3 inhibitor with inert non-toxic pharmaceutically suitable auxiliaries. These auxiliaries include inter alia carriers (for example microcrystalline cellulose, lactose, mannitol), solvents (for example liquid polyethylene glycols), emulsifiers and dispersants or wetting agents (for example sodium dodecyl sulfate, polyoxysorbitan oleate), binders (for example polyvinylpyrrolidone), synthetic and natural polymers (for example albumin), stabilizers (for example antioxidants, such as, for example, ascorbic acid), colorants (for example inorganic pigments, such as iron oxides), and flavor and/or odor corrigents.

The Gal-3 inhibitor may be combined with one or more other active ingredients. The other active ingredients include, for example, tocolytic agents such as ritodrine hydrochloride, isoxsuprine hydrochloride, or magnesium sulfate.

The other active ingredient and therapeutically effective amount thereof may be selected in accordance with techniques and judgement of physicians of ordinal skill. The other active ingredient may be contained together with the Gal-3 inhibitor in a single pharmaceutical preparation or provided separately from the Gal-3 inhibitor.

As used herein, using ingredients "in combination" or "combining" ingredients means not only using a dosage form comprising all the ingredients or using a combination of dosage forms which separately comprise each ingredient, but also administering each ingredient concomitantly, sequentially, or at different times as long as the ingredients are used for reducing a risk of preterm birth and/or low birth weight. Two or more further active ingredients may be used in combination.

The embodiments described herein are non-limiting and may be modified without deviating from the scope of the invention as defined by the appended claims. The following examples do not restrict or limit the invention and are for illustrative purposes only.

### EXAMPLES

### EXAMPLE 1

### Establishment of mouse model of dental infection

The experimental protocol described below was approved by the animal care committee of Hiroshima University. 8-wks-old female C57Bl/6J mice (Charles River Japan, Inc., Yokohama, Japan), with a total number of 52, were kept at constant ambient temperature (22°C) and fed a solid diet ad libitum. Mice were randomly divided into two groups with or without *P.g.* infection and named as *P.g*.-infected group or NC group, respectively. Odontogenic infection of *P.g.* w83 strain was carried out as described before (Furusho, H., et al., Dental infection of Porphyromonas gingivalis exacerbates high fat diet-induced steatohepatitis in mice. J Gastroenterol, November 2013, 48 (11), pp 1259-1270). Briefly, the pulp chambers of the upper first molars in the both side were opened with #1/2 round burr. After removing the coronal pulp, a cotton swab containing 10⁸ CFU of *P.g.* W83 strain was inserted into the pulp chamber, and the pulp chamber was sealed with Caviton (GC Co., Tokyo, Japan). Periapical granuloma was established and level of serum LPS increased at 6wks post-infection. The serum levels of pro-inflammatory cytokines were also measured at 6wks post-infection. Up-regulation of TNF-α, IL-1β, IL-6 and IL-17 was observed, indicating the establishment of systemic low-grade inflammation.

### EXAMPLE 2

### Preterm birth and low birth weight in mouse model of dental infection

Differences in gestational days (gd) and birth weight between NC group and *P.g*.-infected group was studied. In P.g.-infected group mating was started at 6wks post-infection. Gestational days were confirmed by the method of mating once a week. Statistical differences among the experimental groups were evaluated by analysis of T test or chi-square test using GraphPad Prism (version 6.0c, CA, USA) with the level of significance set at P<0.01**.

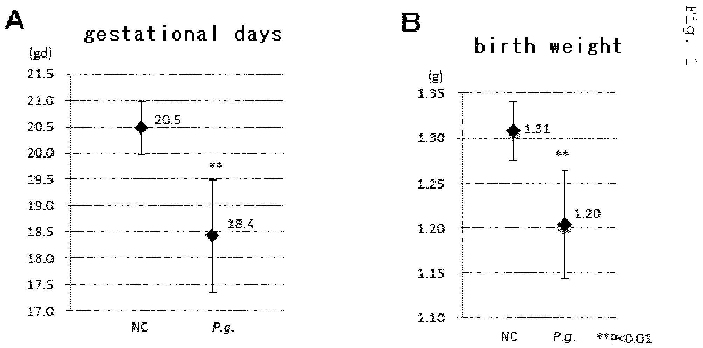

*P.g.* infection didn't affect the maternal body weight. Compared to the average gd of 20.5 in NC mice, *P.g.* infection induced the delivery at as early as gd 18.4 (P<0.01 in T test) in average, which is considered as preterm birth of mice (Table 1). Even some deliveries at gd 17 (very preterm birth of mice) were included. Moreover, the mean birth weight of all the fetuses in *P.g*.-infected group (1.23 g) was significantly lower than that of NC group (1.33 g) (P<0.01 in T test) (Fig. 1B). In addition, low birth weight was observed in P.g.-infected group regardless of the number of fetuses in the litter. For evaluating the birth weights, either birth weight criteria or small-for-gestational age (SGA) criteria (birth weight below the 10th percentile for gestational age) were used (Battaglia FC, Lubchenco LO. A practical classification of newborn infants by weight and gestational age. J Pediatr. 1967 Aug; 71(2):159-63). The birth weight in P.g.-infected group (1.23 g) didn't satisfy the criterion for low birth weight in mouse (<0.93 g), which was calculated from the criterion for low birth weight in human (Table 2). In P.g.-infected group, 30 fetuses (4 litters) out of all 153 fetuses (19.6%) were born at term, including 5 fetuses x 1 litter at gd 19.5; 9 fetuses x 1 litter at gd 19.5; 8 fetuses x 2 litters at gd 20. Among the 30 fetuses, 13 fetuses (43%) were with a birth weight below 10th percentile of NC group (SGA) (P<0.0001 in chi-square test).

**Table 1**

| criteria for preterm birth | | |
|---|---|---|
| | gestational period | |
| | human (weeks) | mouse (days) |
| full-term birth | 40 | 20/21 |
| preterm birth | <37 | <18.5/19.5 |
| very preterm birth | <32 | <16/17 |

**Table 2**

| criteria for low birth weight | | |
|---|---|---|
| | weight of newborn infant | |
| | human (g) | mouse (g) |
| standard weight | 3400 | 1.26 |
| low weight | <2500 | <0.93 |

### EXAMPLE 3

### P.g. detection in P.g-infected placenta

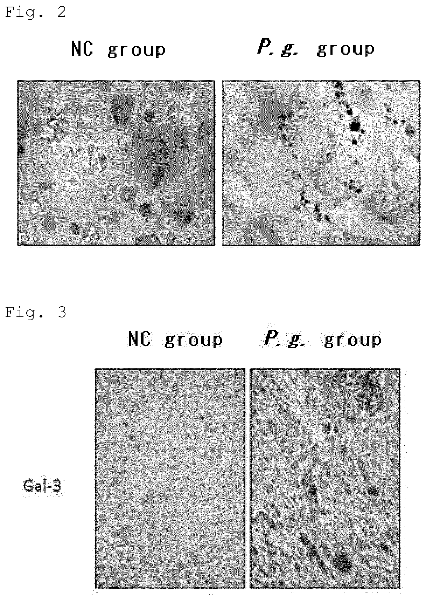

The presence of *P.g.* in placental tissues of *P.g.-*infected group was studied. Immunohistochemical staining was performed in a conventional manner. Immunolocalization of *P.g.* in the placental tissues was studied using a rabbit antiserum against whole *P.g.,* which was kindly gifted from Prof. Kazuyuki Ishihara (Tokyo Dental College, Japan) and Dr. Kazuhisa Ouhara (Hiroshima University, Japan) (1:1,000 dilution). A high polymer method (Histofine Max-PO; Nichirei Biosciences) was used for immunohistochemical detection. Specificity of the antiserum was ascertained by substituting PBS/rabbit serum for the antiserum. *P.g.* colonies were immunohistochemically detected in the P.g-infected placenta (Fig. 2).

### EXAMPLE 4

### Dental infection of P.g. up-regulates inflammatory cell infiltration, inflammatory mediators and Gal-3 in placental tissue

Labor at term involves physiological inflammatory processes such as infiltration of leukocytes and production of proinflammatory cytokines. Localization of inflammatory cells such as polymorphonuclear leukocytes (PMNLs) and macrophages in placenta of gd 15 mice was immunohistochemically studied.

Immunohistochemical staining was performed in a conventional manner. As PMNL markers, MCA771GA (rat anti-neutrophils antibody, purchased from AbD Serotec (Raleigh, NC, USA), 1:5000), rabbit monoclonal anti-COX-2 antibody (purchased from Cell Signaling Technology (Danvers, MA, USA), 1:100), goat anti-TNF-α antibody (purchased from Santa Cruz Biotechnology, Inc. (Santa Cruz, CA, USA), 1:50), and rat anti-galectin-3 antibody (purchased from BioLegend, Inc (San Diego, CA, USA), 1:500) were used. A high polymer method (Histofine Max-PO; Nichirei Biosciences) was used for immunohistochemical staining of the markers. Specificity of the antibodies was ascertained by substituting PBS/serum for each antibody.

Infiltration of inflammatory cells into the placenta was observed in *P.g*.-infected group. PMNLs were restricted in the blood vessel regions in NC group, whereas a lot of PMNLs were also observed in the trophoblast in *P.g.* infected group. Although cells positive for pro-inflammatory mediators, TNF-α and COX-2, could barely be detected in the placenta of NC group, numerous TNF-α and COX-2 positive cells were observed in the placenta of P.g.-infected group.

Interestingly, ubiquitous staining of Gal-3 was detected in the gd 15 placental tissue of *P.g.*-infected group. Unlike in the placenta of NC group, Gal-3 was expressed not only in the inflammatory macrophages but also in the trophoblasts and the endothelial cells (Fig. 3).

### EXAMPLE 5

### Dental infection of P.g. up-regulates Gal-3 in amniotic fluid and serum of mice

Gal-3 level in amnion fluid and serum was studied in NC group and P.g.-infected group.

The amniotic fluid and the serum were collected from gd 15 mice and the Gal-3 levels were measured using mouse Galectin-3 DuoSet ELISA Development Kit from R&D Systems (Minneapolis, MN, USA) according to the manufacturer's instruction. Statistical differences among the experimental groups were evaluated by analysis of T test using GraphPad Prism (version 6.0c, CA, USA) with the level of significance set at P<0.01**.

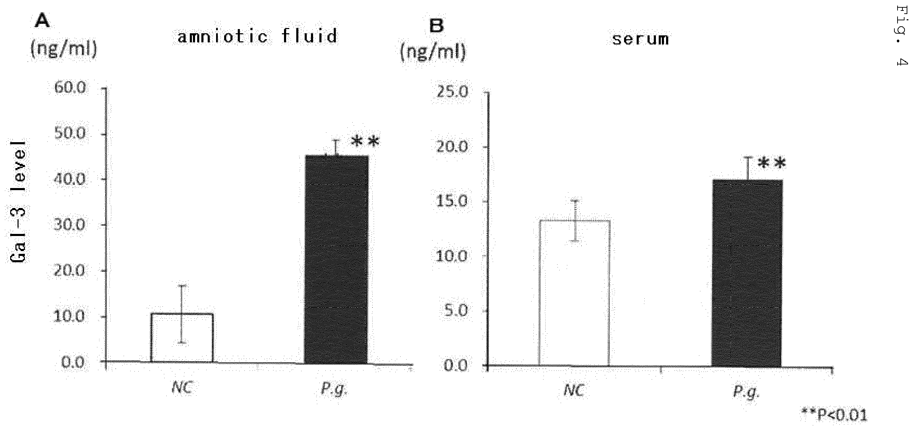

The result is shown in Fig. 4. The Gal-3 levels both in the gd 15 amniotic fluid and serum were significantly higher in *P.g*.-infected group (P<0.01).

### EXAMPLE 6

### P.g.-LPS stimulation up-regulates Gal-3

Alteration in Gal-3 expression by *P.g.*-LPS stimulation was studied in trophoblasts in culture.

Human trophoblast cell line - HTR-8/Svneo cells were kindly gifted from Prof. Charles H. Graham (Queen's University, Canada). The cells were grown in RPMI 1640 media (Nissui Pharmaceutical Co., Tokyo, Japan) supplemented with 10% heat-inactivated FBS (Invitrogen) and 100 Uml penicillin-streptomycin (Gibco, Tokyo, Japan). For the experiments, cells were seeded at a density of 5 × 10⁵ cells in 35 mm culture dishes. The cells were maintained at 37°C in a normal atmosphere containing 5% CO₂. The cells were stimulated by 1 µg/ml of P.g.-LPS (Invivogen, SanDiego, CA, USA), expression of Gal-3 mRNA and Gal-3 levels in the culture media were measured at one, two, or three days after the stimulation.

Total RNAs were extracted from the cell pellets (1.5 x 10⁵ cells) using the RNeasy Mini Kit (Qiagen, Venlo, The Netherlands) following the manufacturer's instructions. The RNA concentration and purity were determined by a standard spectrophotometric method. For cDNA synthesis 1 µg of each total RNA was used with the ReverTra Dash kit (TOYOBO, Osaka, Japan). Aliquots of the total cDNA were amplified with Go Taq (registered trademark)- Green Master Mix (Promega, Madison, WI, USA). Amplification of human Gal-3 gene was performed in a MyCycler™ thermal cycler (BIO-RAD, Tokyo, Japan) for 30 cycles repeating an initial 30 sec denaturation at 94°C, annealing for 30 sec at 58°C, and extension for 1 min at 72°C. GAPDH and 18S were used as internal controls. The products of the amplification reaction were resolved on 1.5% agarose/TAE gels (Nacalai tesque, Inc., Kyoto, Japan), electrophoresed at 100 mV, and visualized by ethidium-bromide staining. The PCR primer pair was
Forward: CTTCCACTTTAACCCACGCTTCAA, and
Reverse: TGTCTTTCTTCCCTTCCCCAGTTATT.

Gal-3 protein levels in the cell culture supernatants were analyzed by Human Galectin-3 Immunoassay (R&D Systems) as the manufacturer recommended. Statistical differences among the experimental groups were evaluated by analysis of T test using GraphPad Prism (version 6.0c, CA, USA) with the level of significance set at P<0.01**.

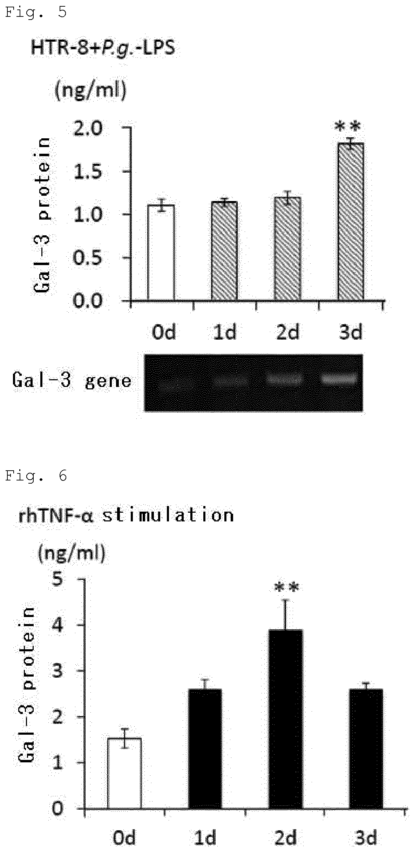

The result is shown in Fig. 5. The P.g.-LPS stimulation up-regulated both the mRNA expression and the protein level of Gal-3 at day 3 (P<0.01).

### EXAMPLE 7

### TNF-α stimulation up-regulates Gal-3

Effect of TNF-α, which is produced by *P.g.*-LPS stimulation, on Gal-3 production was studied.

Recombinant human TNF-α (rhTNF-α, purchased from R&D Systems (Minneapolis, MN, USA)) was applied at 10 ng/ml to HTR-8 cells for 1, 2 or 3 days and the Gal-3 protein levels in the culture media were measured. The cell culture and measurement of the Gal-3 protein levels were performed in the same manner as in Example 6.

The result is shown in Fig. 6. The Gal-3 level was significant increased at 2 days post-stimulation (P<0.01).

### EXAMPLE 8

### Comparison of serum Gal-3 levels between pregnant women with full-term birth and preterm birth

Correlation between preterm birth and Gal-3 expression was studied in human.

Blood samples were obtained from pregnant women with full-term birth at the gestational ages of 12 weeks (n=2), 28 weeks (n=2), 32 weeks (n=2), and 38 weeks (n=3), and from pregnant women with preterm birth (n=2) immediately before the delivery, and serum samples were prepared from the blood samples. In the preterm birth group the average gestational age at the delivery was 33 weeks. Gal-3 levels in the serum samples were measured by Human Galectin-3 DuoSet ELISA Development Kit (R&D Systems, Minneapolis, MN, USA) as the manufacturer recommended. Statistical differences among the experimental groups were evaluated by analysis of T test using GraphPad Prism (version 6.0c, CA, USA) with the level of significance set at P<0.05.

The result is shown in the table below and Fig. 7.

**[Table 3]**

| full-term birth /preterm birth | gestational age (wks) | serum Gal-3 level (ng/ml) |
|---|---|---|
| full-term birth | 38 | 14.96 |
| full-term birth | 38 | 32.29 |
| full-term birth | 38 | 25.83 |
| full-term birth | 12 | 9.80 |
| full-term birth | 12 | 10.53 |
| full-term birth | 28 | 5.98 |
| full-term birth | 28 | 13.04 |
| full-term birth | 32 | 14.54 |
| full-term birth | 32 | 14.26 |
| | | |
| preterm birth | 29 | 61.36 |
| preterm birth | 35 | 28.91 |
| preterm birth | 33 | 34.62 |
| preterm birth | 31 | 22.65 |
| preterm birth | 36 | 46.22 |
| preterm birth | 36 | 43.35 |

In the full-term birth group the Gal-3 levels were almost constant at the gestational ages of 12, 28 and 32 weeks, being 11.36 ng/ml (± 3.27 SD) on average. The Gal-3 levels were increased to 24.36 ng/ml (± 8.76 SD) on average at the gestational age of 38 weeks (Fig.s 7A and 7B). The average Gal-3 level immediately before the delivery was 39.52 ng/ml (± 13.84 SD) in the preterm birth group, being higher than that in the full-term birth group. Even though the average gestational period was 33 weeks in the preterm birth group, the average Gal-3 level was higher than that of the full-term birth group at the gestational age of 38 weeks (P<0.05) (Fig. 7C).

### EXAMPLE 9

### Serum Gal-3 levels in pregnant women

Similarly to Example 8, the serum Gal-3 levels in pregnant women were measured at the gestational ages from 11 to 40 weeks. The result is shown in the table below and Fig. 8. The result includes the values measured for the full-term birth group in Example 8, which are indicated by the symbol *. Values without the symbol mean that the women have not delivered yet, thus the values without the symbol at the gestational ages from 11 weeks to 37 weeks cannot be assigned to either of the preterm birth group or the full-term birth group. The result also includes the values measured for the preterm birth group in Example 8 (PTB), which are indicated by the symbol **.

**[Table 4]**

| gestational period (wks) | 11-19W | 20-30W | 30-37W | 38-40W | PTB |
|---|---|---|---|---|---|
| serum Gal-3 level (ng/ml) | 9. 62 | 11.35 | 20.85 | 14.96* | 61.36** |
| | 9. 68 | 10.18 | 14.54* | 32.29* | 28.91** |
| | 9.96 | 12.41 | 14.26* | 25.83* | 34.62** |
| | 10.08 | 19.63 | 9.81 | 26.58 | 22.65** |
| | 16.79 | 9.81 | 11.21 | | 46.22** |
| | 9.8* | 10.00 | 30.01*** | | 43.35** |
| | 10.53* | 11.57 | 12.32 | | |
| | 9.54 | 5.98* | 9.74 | | |
| | 9.82 | 13.04* | 11.34 | | |
| | 10.14 | 9.51 | 9.51 | | |
| | 10.14 | 9.54 | 18.14 | | |
| | 10.66 | 9.72 | 9.58 | | |
| | 11.65 | 17.28 | | | |
| | 13.16 | 26.32 | | | |
| | | 38.47*** | | | |
| | | 9.75 | | | |
| | | 37.72*** | | | |
| average | 10.83 | 15.43 | 14.28 | 24.92 | 39.52 |
| SD | 1.97 | 9.77 | 6.13 | 7.24 | 13.84 |
| SE | 0.53 | 2.37 | 1.77 | 3.62 | 5.65 |

The serum Gal-3 levels varied from about 10 to about 15 ng/ml at the gestational ages from 11 weeks to 37 weeks, and increased to from 25 to 30 ng/ml at the gestational age around 38 weeks. The average serum Gal-3 level at the gestational ages from 11 weeks to 37 weeks was 13.70 ng/ml (± 7.23 SD). Some values were extremely higher than the average, in particular, the values indicated by the symbol *** were higher than the average + 2σ. When these values are excluded, the average serum Gal-3 level at the gestational ages from 11 weeks to 37 weeks was 12.21 ng/ml (± 3.90 SD).

### EXAMPLE 10

### Effect of Gal-3 inhibitor on preterm birth and/or low birth weight

Effect of a Gal-3 inhibitor, N-acetyllactosamine (N-Lac), on preterm birth induced by dental infection of *P.g.* was studied.

8-wks-old female C57Bl/6J mice were infected with *P.g.* w83 strain via the pulp chambers of the first molars. After periapical granuloma, which serves as a persistent source of *P.g.,* was established at 6wks post-infection, mating was started (*P.g.* group). A half of the mice in *P.g.* group was intraperitoneally administered with 5 mg/kg of N-Lac (Sigma-Aldrich) at the gestational ages of 12, 15 and 18 days (*P.g*./N-Lac group). Healthy pregnant mice without *P.g.* infection were used as a control (NC group). The gestational period was compared among the groups. At the gestational age of 17 days the placental tissues were harvested, and inflammatory cell infiltration (MΦ, neutrophils), damage of endothelial cells (CD31), and immunolocalization of factors promoting delivery (TNF-α, COX-2, and Gal-3) were compared among the groups.

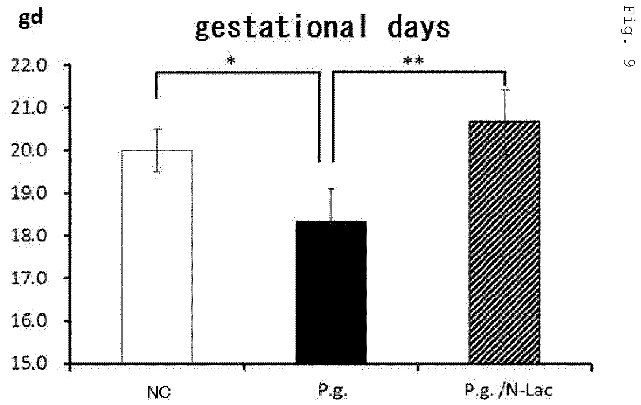

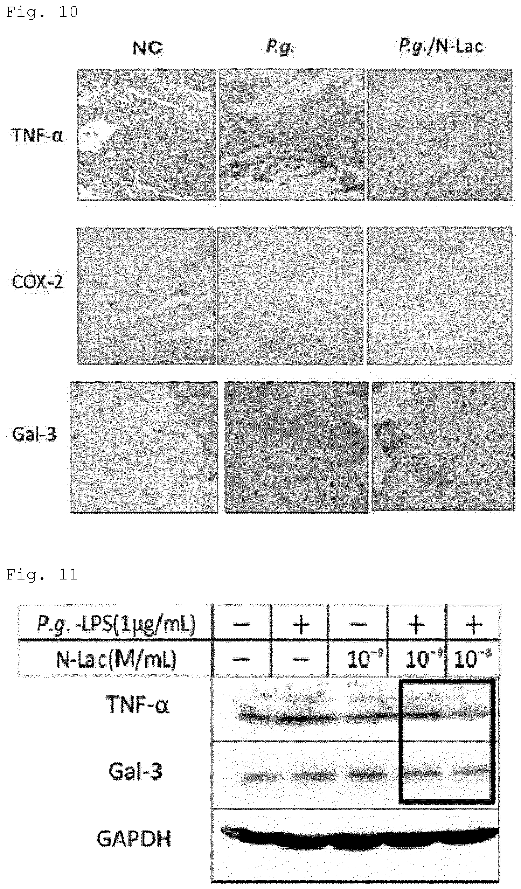

As shown in Fig. 9, the average gestational period was 20, 18.3, and 20.7 days in NC, *P.g.,* and *P.g*./N-Lac groups, respectively. The administration of N-Lac significantly suppressed preterm birth induced by *P.g.* (p<0.05). The histological observation of the placenta in *P.g.* group at the gestational age of 17 days revealed denature and detachment of the amnion membrane (sign of early amniorrhexis), denature and necrosis of the placenta, and increase of inflammatory cell infiltration, but they are not observed in *P.g*./N-Lac group. Decrease of CD31 and expression of TNF-α, COX-2 and Gal-3 were observed in *P.g.* group, but improved in *P.g*./N-Lac group (Fig. 10).

### EXAMPLE 11

### Effect of Gal-3 inhibitor on human placenta cell line

HTR-8 cells were stimulated with *P.g.-*LPS (1 µg/ml) and N-Lac (10⁻⁹ M and 10⁻⁸ M), and the expression levels of Gal-3, TNF-α, COX-2 and IL-8 in the culture supernatants were measured by RT-PCR and Western-blotting.

The result of the Western-blotting is shown in Fig. 11. The incubation with N-Lac suppressed the expression of TNF-α, which promotes Gal-3, and remarkably reduced the TNF-α and Gal-3 expression upregulated by the *P.g.*-LPS stimulation.

### INDUSTRIAL APPLICABILITY

According to the disclosure above, a risk of preterm birth and/or low birth weight can be determined. The result is useful in the selection of pregnant females to be subjected to the prevention of preterm birth and/or low birth weight, promoting effective and efficient perinatal care. Additionally, a risk of preterm birth and/or low birth weight may be reduced by a Gal-3 inhibitor.

## Claims

1. A method for determining a risk of preterm birth and/or low birth weight of a pregnant woman comprising measuring a Gal-3 level in a blood, plasma, or serum sample obtained from the pregnant woman.

2. The method according to claim 1, wherein the Gal-3 level is compared with a reference value and the pregnant woman is determined to have a risk of preterm birth and/or low birth weight when the Gal-3 level is higher than the reference value.

3. The method according to claim 2, wherein the reference value is a value determined on the basis of an average Gal-3 level at the time of delivery in a group of pregnant women with full-term birth.

4. The method according to any one of claims 1 to 3, wherein the pregnant woman is determined to have a risk of preterm birth and/or low birth weight when the pregnant woman is at a gestational age not more than 36 weeks and 6 days and the Gal-3 level is not less than 13 ng/ml.

5. The method according to any one of claims 1 to 4, wherein the pregnant woman is determined to have a high risk of preterm birth and/or low birth weight when the pregnant woman is at a gestational age not more than 36 weeks and 6 days and the Gal-3 level is not less than 18 ng/ml.

6. The method according to any one of claims 1 to 5, wherein the pregnant woman is determined to have a very high risk of preterm birth and/or low birth weight when the pregnant woman is at a gestational age not more than 36 weeks and 6 days and the Gal-3 level is not less than 25 ng/ml.

7. The method according to any one of claims 1 to 6, wherein the Gal-3 level is measured by using an anti-Gal-3 antibody.

8. The method according to any one of claims 1 to 7, wherein the pregnant woman has a risk factor of preterm birth and/or low birth weight.

9. The method according to claim 8, wherein the risk factor is multiple pregnancy, previous preterm birth or miscarriage, lack of prenatal care, poor nutrition, stress, early or advanced maternal age pregnancy, smoking, diabetes, high blood pressure, an infectious disease, or an uterine or cervical problem.

10. The method according to any one of claims 1 to 9, wherein the pregnant woman is suffered from periodontitis caused by periodontopathic bacteria.

11. The method according to claim 10, wherein the periodontopathic bacteria is *Porphyromonas gingivalis.*

12. A kit for determining a risk of preterm birth and/or low birth weight of a pregnant woman comprising an anti-Gal-3 antibody for measuring a Gal-3 level in a blood, plasma, or serum sample obtained from the pregnant woman.

13. Use of Gal-3 in a blood, plasma, or serum sample obtained from a pregnant woman as a marker of a risk of preterm birth and/or low birth weight of the pregnant woman.

14. A pharmaceutical composition for reducing a risk of preterm birth and/or low birth weight comprising a Gal-3 inhibitor as an active ingredient.

15. A method for reducing a risk of preterm birth and/or low birth weight of a pregnant woman comprising administering an effective amount of a Gal-3 inhibitor to the pregnant woman.


SEQUENCE LISTING

<110>  Hiroshima University

<120>  METHOD FOR DETERMINING RISK OF PRETERM BIRTH AND/OR LOW BIRTH
       WEIGHT AND KIT THEREFOR

<130>  672450

<150>  JP 2014-165280
<151>  2014-08-14

<160>  2

<170>  PatentIn version 3.5

<210>  1
<211>  24
<212>  DNA
<213>  Artificial Sequence

<220>
<223>  forward primer for amplifying human galectin-3

<400>  1
cttccacttt aacccacgct tcaa                                              24


<210>  2
<211>  26
<212>  DNA
<213>  Artificial Sequence

<220>
<223>  reverse primer for amplifying human galectin-3

<400>  2
tgtctttctt cccttcccca gttatt                                            26